**Europäisches Patentamt**

**European Patent Office**

**Office européen des brevets**

(19)

(11) Veröffentlichungsnummer: **0 186 762**
**B1**

(12) # EUROPÄISCHE PATENTSCHRIFT

(45) Veröffentlichungstag der Patentschrift:
**26.07.89**

(21) Anmeldenummer: **85114342.0**

(22) Anmeldetag: **12.11.85**

(51) Int. Cl.⁴: **A 61 M 1/00**

(54) **Vorrichtung zum Absaugen von Sekretflüssigkeit aus einer Wunde.**

(30) Priorität: **16.11.84 DE 3441893**

(43) Veröffentlichungstag der Anmeldung:
**09.07.86 Patentblatt 86/28**

(45) Bekanntmachung des Hinweises auf die Patenterteilung:
**26.07.89 Patentblatt 89/30**

(84) Benannte Vertragsstaaten:
**AT BE CH DE FR GB IT LI LU NL SE**

(56) Entgegenhaltungen:
**EP-A- 0 100 672**
**EP-A- 0 113 541**
**DD-A- 124 498**
**DE-A- 2 359 338**
**DE-B- 2 108 757**
**US-A- 3 565 076**

(73) Patentinhaber: **Beck, Walter, Häsligenstrasse 8,
CH-8700 Küsnacht (CH)**
Patentinhaber: **Werner, Margrit,
Philipp-Wasserburgstrasse 30,
D-6500 Mainz-Gonsenheim (DE)**

(72) Erfinder: **Beck, Walter, Maybachstrasse 11,
D-7022 Leinfelden-Echterdingen (DE)**
Erfinder: **Werner, Margrit, Dipl.-Ing.,
Philipp-Wasserburg-Strasse 30,
D-6500 Mainz-Gonsenheim (DE)**

(74) Vertreter: **Patentanwälte Phys. Bartels Dipl.-Ing. Fink
Dr.-Ing. Held, Lange Strasse 51, D-7000 Stuttgart 1 (DE)**

## Beschreibung

Die Erfindung betrifft eine Vorrichtung zum Absaugen von Sekretflüssigkeit aus einer Wunde, die die Merkmale des Oberbegriffs des Anspruchs 1 aufweist.

Bei der postoperativen Absaugung von Sekretflüssigkeit aus einer Wunde ist es für die optimale Förderung des Heilungsprozesses notwendig, dass der am in die Wunde eingeführten Drain wirkende Unterdruck im gesamten Zeitraum vom Beginn bis zum Ende des Absaugvorganges entsprechend den hierdurch sich ergebenden Erfordernissen frei gewählt werden kann. Auch kann durch die richtige Wahl des Unterdruckes beim Entfernen des Drains eine wesentliche Reduzierung der Gewebeschädigung in der Wunde erreicht werden. Im Gegensatz zu einer Verstärkung des Unterdruckes, die üblicherweise durch eine Aktivierung einer Pumpe bewirkt werden kann, ist eine Verminderung des Unterdruckes durch ein entsprechendes Ansteuern der Pumpe nicht möglich.

Bei einer bekannten Vorrichtung der oben genannten Art (EP-A-0 113 541) wird der am Drain wirkende Unterdruck dadurch vermindert, dass ein Regler abhängig von dem vorgegebenen Sollwert den Querschnitt einer Drossel, die über entsprechende Schläuche die Umgebung mit dem Drain verbindet, derart vergrössert, dass Luft aus der Umgebung mit dem dort herrschenden Luftdruck so lange in das aus Drain und Schläuchen gebildete System nachströmt, bis der über eine Druckmesseinrichtung abgegriffene Istwert des Druckes im System dem vorgegebenen Sollwert entspricht. Bei dieser bekannten Vorrichtung zur Regelung des momentan im System herrschenden Unterdruckes zu einem vorgegebenen Sollwert hin, kann also notwendig sein, Luft von aussen her in den zum Drain führenden Teil der Schläuche eintreten zu lassen, was ein Sterilhalten derjenigen Teile, die dem Unterdruck ausgesetzt sind, ausschliesst und somit die Infektionsgefahr erhöht.

Der Erfindung liegt die Aufgabe zugrunde, eine Vorrichtung zu schaffen, die nicht nur eine Verstärkung, sondern auch ein Vermindern des Unterdruckes ermöglicht, ohne dass damit eine Infektionsgefahr verbunden ist. Diese Aufgabe löst eine Vorrichtung mit den Merkmalen des Anspruches 1.

Bei der erfindungsgemässen Vorrichtung wird der am Drain wirkende Unterdruck durch eine Verringerung des Innenvolumens, in dem dieser Unterdruck herrscht, vermindert, indem das Innenvolumen des mit dem Drain über einen Schlauch verbundenen Hohlkörpers verringert wird. Der im System herrschende Unterdruck kann somit vermindert werden, ohne dass von aussen Luft hinzutreten müsste. Das System kann deshalb ständig mikrobiologisch geschlossen ausgebildet sein, was eine Infektionsgefahr für die Wunde ausschliesst.

Bei der erfindungsgemässen Vorrichtung wäre es zwar auch möglich, die Volumenänderung durch eine Person vornehmen zu lassen. Vorteilhafterweise ist jedoch der Hohlkörper mit einer vom Regler steuerbaren Antriebseinrichtung versehen. Der Einsatz eines Reglers ist sowohl im Hinblick auf die relativ hohen Personalkosten als auch im Hinblick auf die Ausschaltung von auf menschliches Versagen zurückzuführende Fehlerquellen vorteilhaft.

Im Hinblick auf di erforderliche Sterilität sind vorzugsweise alle diejenigen Teile des geschlossenen Systemes, welche dem Unterdruck ausgesetzt sind, als Wegwerfteile ausgebildet.

Die Vorgabe des Unterdruckes in Form vo Sollwerten ist in verschiedener Weise möglich. So kann die Sollwertvorgabe in digitaler oder analoger Form erfolgen. Es besteht damit die Möglichkeit, ein an die Wundverhältnisse und ein an die einzelnen Umstände angepasstes Druck-Zeit-Diagramm als Sollwertvorgabe zu erstellen. Man kann dann ein derartiges Diagramm beispielsweise in einem Speicher oder auch einem Datenträger festlegen und bei Bedarf abrufen. Auch hier ist die Einhaltung des Druck-Zeit-Diagrammes, auf das der jeweils im System herrschende Druck-Istwert einregelbar ist, nicht von der Aufmerksamkeit oder von der Zuverlässigkeit des Personals abhängig, was vor allem im postoperativen Bereich wichtig ist.

Besonders vorteilhaft ist hierbei, die Unterdruckquelle durch einen Abschnitt des vom Drain zum Sammelbehälter führenden Schlauches zu bilden, welcher der Einwirkung einer Schlauchpumpe aussetzbar ist. Der Regler kann hierbei die Drehzahl des auf den Schlauchabschnitt einwirkenden Kopfes der Schlauchpumpe im Bereich zwischen der Drehzahl 0 und der maximalen Drehzahl regeln. Dennoch ist die Herstellung der Verbindung zwischen dem vom Drain zum Sammelbehälter führenden Schlauch mit dem Pumpenkopf und das Lösen der Verbindung so einfach, dass die Handhabung des Systems hierdurch für das Personal nicht erschwert wird.

Damit die Unterdruck-Messeinrichtung einen möglichst geringen Kostenaufwand verursacht, ist sie bei einer bevorzugten Ausführungsform in einen Sensorteil und einen Auswerteteil unterteilt. Der Sensorteil ist hierbei vorzugsweise ein aus Kunststoff bestehender Wegwerfteil mit zwei gegeneinander wirkenden Membranen unterschiedlicher Flächengrösse. Hierdurch kann in einfacher Weise die Relativbewegung der beiden Membranen in eine von aussen her aufnehmbare Druckkraft umgesetzt werden, was eine wesentlich einfachere Kopplung an den Auswerteteil erlaubt, als dies der Fall wäre, wenn eine Zugkraft aufgenommen werden müsste. Vorzugsweise ist in einem Gehäuse, das den Auswerteteil der Unterdruck-Messeinrichtung, aber auch andere Komponenten der Vorrichtung, zum Beispiel den Kopf der Schlauchpumpe und deren Antriebsmotor sowie den Regler enthalten kann, ein Halter für den Sensorteil vorgesehen, in den letzterer nur eingesetzt oder eingeschoben zu werden braucht, um ihn mit dem Auswerteteil zu koppeln. Dadurch wird ebenfalls die Handhabung des vorzugsweise geschlossenen Systems nicht nennenswert erschwert.

Der Hohlkörper mit veränderbarem Innenvolumen ist im Hinblick auf die erforderliche Sterilität und eine Realisierbarkeit als Teil eines geschlossenen Wegwerfsystems vorzugsweise durch einen Faltenbalg gebildet, dessen beide stirnseitigen Begrenzungswände mittels einer Antriebsvorrichtung in Balglängsrichtung relativ zueinander verstellbar sind. Für die üblicher Weise erforderlichen Volumina lässt sich ein derartiger Faltenbalg aus Kunststoff mit relativ geringen Kosten herstellen. Es braucht deshalb nur dafür Sorge getragen zu werden, dass die Kupplung mit seiner Antriebsvorrichtung so ausgebildet ist, dass sie ohne Schwierigkeiten in Eingriff und ausser Eingriff gebracht werden kann. Hierzu ist es zweckmässig, eine Halterung, beispielsweise in dem auch die übrigen Komponenten enthaltenen Gehäuse vorzusehen.

Im folgenden ist die Erfindung anhand eines in der Zeichnung dargestellten Ausführungsbeispiels der erfindungsgemässen Vorrichtung im einzelnen erläutert. Es zeigen

Fig. 1 eine schematische Darstellung des Ausführungsbeispiels,

Fig. 2 eine Draufsicht auf ein verschiedene Komponenten enthaltendes Gehäuse,

Fig. 3 einen Längsschnitt durch den Sensorteil einer Unterdruck-Messeinrichtung

Fig. 4 einen Längsschnitt durch den Hohlkörper veränderbaren Volumens und séine Antriebsvorrichtung

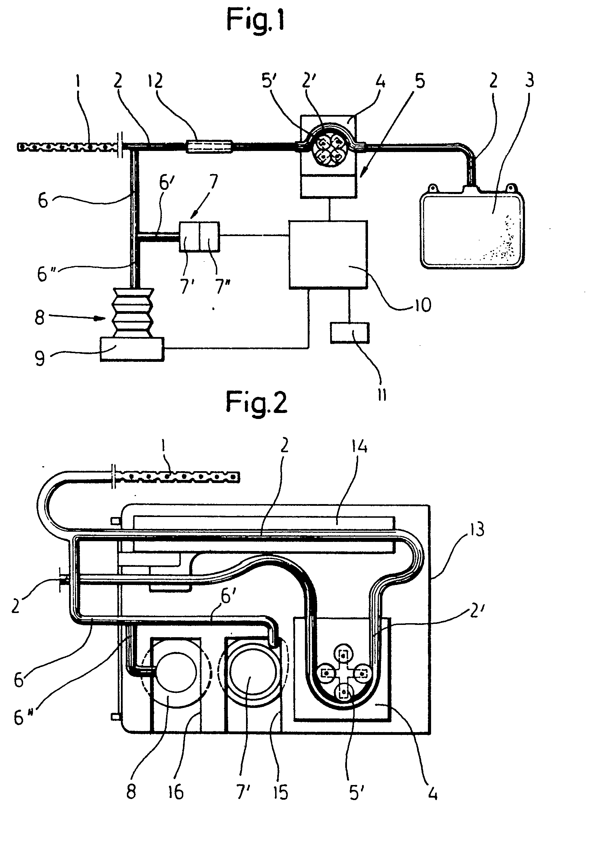

Wie in Figur 1 zeigt, ist ein Drain 1 über einen Schlauch 2 mit einem Sammelbehälter 3 verbunden, der die aus der Wunde abgesaugte Sekretflüssigkeit aufnimmt. Der Schlauch 2 weist einen Abschnitt 2' auf, der in eine Aufnahme 4 legbar ist, in die der drehbare Kopf einer in bekannter Weise ausgebildeten und daher im einzelnen nicht dargestellten Schlauchpumpe 5 ragt. Der Kopf der Schlauchpumpe 5 ist mit drehbar gelagerten Rollen 5' versehen, die sich, so lange sie während der Rotation unter Druck am Abschnitt 2' anliegen, auf diesem abwälzen. Als Antrieb für die Schlauchpumpe 5 dient ein Elektromotor mit regelbarer Drehzahl.

Mit dem Schlauch 2 ist ein zweiter Schlauch 6 verbunden, der sich verzweigt. Der erste Zweig 6' führt zu einem Sensorteil 7' einer Unterdruck-Messeinrichtung 7, der zweite Zweig 6" zu einem als ganzes mit 8 bezeichneten Hohlkörper veränderbaren Volumens, dessen Inneres deshalb mit dem Innenraum des Schlauches 2 in Verbindung steht.

Der Hohlkörper 8 ist über eine lösbare Verbindung mit einer Antriebsvorrichtung 9 verbunden, mittels deren das Volumen des Hohlkörpers 8 auf unterschiedliche Werte einstellbar ist. Im Ausführungsbeispiel beträgt das maximale Volumen des Hohlkörpers 8 etwa 20 ccm. Selbstverständlich kann das Volumen auch entsprechend den Erfordernissen grösser oder kleiner gewählt sein.

Der Drain 1, die beiden Schläuche 2 und 6, der Sammelbehälter 3, der Sensorteil 7' und der Hohlkörper 8 bilden ein in mikrobiologischer Hinsicht geschlossenes System, d.h. während der gesamten Gebrauchsdauer dieses Teils der erfindungsgemässen Vorrichtung können von aussen her keine Erreger in das Innere des Systems gelangen.

Die Drehzahl der Schlauchpumpe 5 wird im Bereich zwischen 0 und der maximalen Umdrehungszahl von einem Regler 10 geregelt, und zwar derart, dass der Istwert des Unterdruckes im zweiten Schlauch 6, der gleich dem Istwert des Unterdruckes ist, der am Drain 1 wirksam ist, zu jedem Zeitpunkt mit dem entsprechenden Sollwert übereinstimmt, der durch ein Druck/Zeit-Diagramm vorgegeben ist. Hierzu erhält der Regler von einem Auswerteteil 7" der Unterdruck-Messeinrichtung, der lösbar mit dem Sensorteil 7' gekoppelt ist, elektrische Signale, die den Istwert repräsentieren. Entsprechend erhält der Regler 10 den Sollwert des Unterdruckes von einem Sollwertgeber 11. Bei diesem Sollwertgeber handelt es sich im Ausführungsbeispiel um einen Speicher, aus dem die einzelnen Sollwerte zeitrichtig ausgelesen werden können. Das Sollwert-Programm könnte aber selbstverständlich auch in anderer Weise zusammengestellt werden, beispielsweise auf einer Magnetkarte, welche in eine Leseeinrichtung des Sollwertgebers eingesteckt wird.

Mit dem Regler 10 ist auch die Antriebsvorrichtung 9 verbunden, die vom Regler im Sinne einer Volumenverringerung aktiviert wird, wenn das Druck/Zeit-Diagramm eine Verminderung des Unterdruckes vorschreibt. Sofern dieses Diagramm mehrere Un Unterdruckverminderungsvorgänge beinhaltet, muss der Regler 10 bei der ersten auf eine Absenkung des Unterdruckes folgenden Unterdruckerhöhung die Antriebsvorrichtung im Sinne einer Volumenvergrösserung aktivieren.

Im Ausführungsbeispiel ist der Schlauch 2 im Bereich zwischen dem Abschnitt 2' und dem Drain 1 in einen Sekretflusssensor 12 einlegbar. Dieser Sensor 12 ist mit einer in Figur 1 als Teil des Reglers 10 dargestellten Auswertelogik verbunden, mit der auch der Ausgang des Reglers 10 für die Schlauchpumpe 5 sowie der Auswerteteil 7" der Unterdruck-Messeinrichtung 7 in Verbindung steht. Die Auswertelogik erzeugt ein Signal, wenn die Schlauchpumpe 5 den Unterdruck im Schlauch 2 nicht über einen vorgegebenen Wert steigern kann, obwohl sie mit maximaler Drehzahl läuft, oder wenn diese Schlauchpumpe 5 mit einer über einem vorgegebenen Grenzwert liegenden Drehzahl läuft und der Sekretflusssensor 12 keinen Sekretfluss meldet. In beiden Fällen ist dies ein Anzeichen für eine Leckage, beispielsweise eine nicht dicht verschlossene Wunde.

Das in Figur 2 dargestellte Gehäuse 13 enthält die Schlauchpumpe 5, den Auswerteteil 7" der Unterdruck-Messeinrichtung 7, die Antriebsvorrichtung 9 für den Hohlkörper 8, den Regler 10, die Auswertelogik, den Sollwertgeber 11 und den Sekretflusssensor 12 sowie einen Energieversorgungsteil. An eine der Gehäusewände, im Ausführungsbeispiel dei Gehäuseoberseite, ist die Aufnahme 4 angeformt, in die der Abschnitt 2' des Schlauches 2 eingelegt wird und in die der Kopf der Schlauchpumpe 5 mit den Rollen 5' ragt. Ferner bildet die Oberseite des Gehäuses 13 einen Halter 14 für den Sekretflusssensor 12, damit der vom Drain 1 zum Abschnitt 2' führende Teil des Schlauches 2 nur in diesen Halter 14 eingelegt zu werden braucht, um den Sekretfluss ermitteln zu können. Schliesslich ist die Oberseite des Gehäuses 13 mit je einer Aufnahme 15 bzw. 16 für den Sensorteil 7' der Unterdruck-Messeinrichtung 7 bzw. den Hohlkörper 8 versehen.

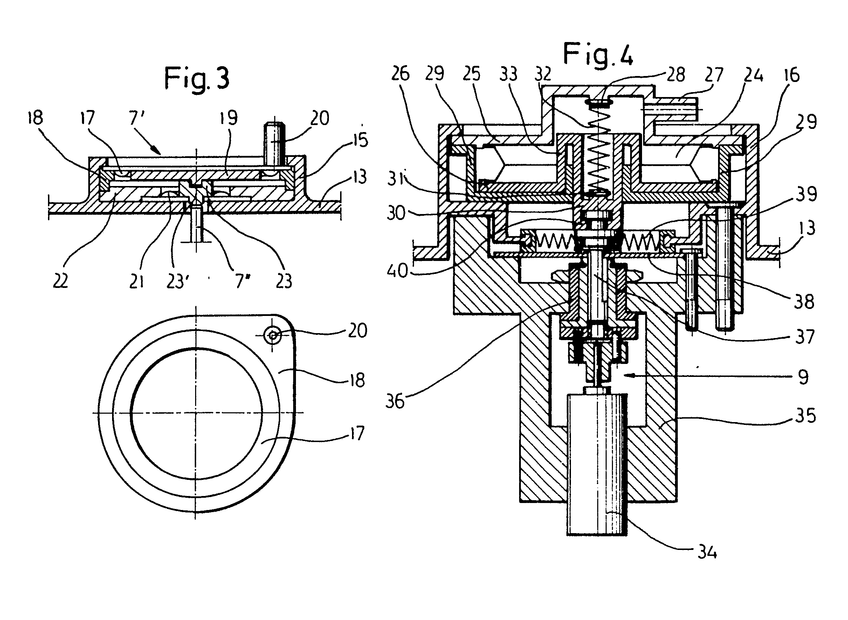

Wie Figur 3 zeigt, weist der nur aus Kunststoffteilen bestehende Sensorteil 7' eine erste Membrane 17 auf, deren äusserer Rand mit einem Ringteil 18 fest verbunden oder einstückig ausgebildet ist. An der Innenseite des Zentralbereichs der ersten Membrane 17 liegt eine ringscheibenförmige Versteifungsplatte 19 an, die aber auch einstückig mit der ersten Membrane 17 ausgebildet sein kann. In diesem Falle kann der ringscheibenförmige Membranteil durch einen dünnen Bereich zwischen der Versteifungsplatte 19 und dem Ringteil 18 gebildet sein. An letzteren ist ein Schlauchanschlussstutzen 20 angeformt, dessen Bohrung mit dem Inneren des Sensorteils 7' in Verbindung steht. Parallel und im Abstand zur ersten Membrane 17 liegt eine zweite Membrane 21, deren Durchmesser wesentlich kleiner ist als derjenige der ersten Membrane 17. Der äussere Rand der zweiten Membrane 21 wird von einem ringscheibenförmigen, formstabilen Halter 22 gehalten. Mit diesem Halter 22, der mit dem Ringteil 18 fest und dicht verbunden ist, kann die Membrane 21 auch einstückig ausgebildet sein. An der Innenseite des zentralen Bereiches der Membrane 21 liegt ein zylindrischer Distanzkörper 23 an, der ein zentrales, zur Versteifungsplatte 19 hin offenes Sackloch aufweist, in das ein zentral angeordneter Zapfen der Versteifungsplatte 19 eingreift. Ferner weist der Distanzkörper 23, der dicht mit der zweiten Membrane 21 verbunden ist, einen eine zentrale Öffnung der zweiten Membrane 21 durchdringenden Stössel 23' auf. Selbstverständlich könnte auch der Distanzkörper 23 einstückig mit der zweiten Membrane 21 ausgebildet sein.

Infolge der stark unterschiedlichen Flächen der beiden Membrane 17 und 21 bewirkt eine zunehmende Evakuierung des Sensorteils 7', dass der Stössel 23' in einer Richtung bewegt wird, in der sein Überstand über die Aussenseite des Halters 22 zunimmt. Die Druckkraft des Stössels 23' und seine Längsverschiebung wird vom Auswerteteil 7'', der einen am Stössel 23' anliegenden Taster hat, in ein die Höhe des Unterdruckes repräsentierendes elektrisches Signal umgewandelt.

Um den Sensorteil 7' mit dem Auswerteteil 7'' zu kuppeln, braucht nur der Sensorteil 7' in die zugehörige Aufnahme 15 des Gehäuses 13 eingeschoben zu werden, welche eine Auflage für die Aussenseite des Halters 22 bildet und den Ringteil 18 übergreift. Ebenso braucht nach Beendigung der Absaugung nur der Sensorteil 7' aus der Aufnahme 15 herausgenommen zu werden.

Prinzipiell gleich ist die Aufnahme 16 ausgebildet, in welche der Hohlkörper 8 eingeschoben wird, um ihn mit seiner Antriebsvorrichtung 9 zu kuppeln.

Wie Figur 4 zeigt, weist der Hohlkörper 8 einen aus Kunststoff oder Gummi bestehenden Faltenbalg 24 auf, der durch einen oberen Verschlusskörper 25 und einen unteren Verschlusskörper 26 dicht verschlossen ist. Der obere Verschlusskörper 25 hat, wie Figur 4 zeigt, die Form einer Platte mit einer nach aussen vorspringenden, nur nach innen offenen Nabe. An diese Nabe ist seitlich ein Anschlussstutzen 27 und innen ein zentral angeordneter Zapfen 28 angeformt. Der äussere Rand des oberen Verschlusskörpers 25 ist mit einem Ringflansch eines wie die Verschlusskörper aus Kunststoff bestehenden Führungskörpers 29 fest und dicht verbunden. Dieser Ringflansch schliesst sich an einen zylindrischen Teil des Führungskörpers 29 an, welcher den Faltenbalg 24 im Abstand konzentrisch umgibt. Der zylindrische Teil erstreckt sich von einem ringscheibenförmigen Teil aus, der parallel zum oberen Verschlusskörper 25 liegt, gegen letzteren hin. In der gleichen Richtung erstreckt sich ein an den ringscheibenförmigen Teil angeformter, zentraler Nabenteil, in dem der untere Verschlusskörper 26 längsverschiebbar geführt ist, und zwar mittels eines diesen Nabenteil durchgreifenden Hohlzapfens 30, dessen nach aussen weisendes Ende verschlossen ist.

An die Innenseite der diesen Verschluss bildenden Materialpartie ist ein gegen den Zapfen 28 weisender Zapfen 31 angeformt. Über beide Zapfen 28 und 31 ist das eine bzw. das andere Ende einer vorgespannten Schraubendruckfeder 32 gesteckt, welche den Faltenbalg 24 im Sinne einer Verlängerung belastet. Der Hohlzapfen 30 ist an seinem inneren Ende an eine ihn im Abstand umgebende Hülse 33 angeformt, die längsverschiebbar in dem Nabenteil des oberen Verschlusskörpers 25 geführt und einstückig mit dem formstabilen, ringscheibenförmigen Teil des unteren Verschlusskörpers 26 ausgebildet ist, der in axialer Richtung verschiebbar zwischen dem Nabenteil und hohlzylindrischen Teil des Führungskörpers 29 liegt.

Die Antriebsvorrichtung 9 des Hohlkörpers 8 ist mit einem in seiner Drehrichtung umkehrbaren Elektromotor 34 ausgerüstet, der gleichachsig zum Hohlzapfen 30 in einer Halterung 35 des Gehäuses 13 angeordnet und mit einer in der Halterung 35 drehbar, aber axial unverschiebbar gelagerten Buchse 36 gleichachsig gekuppelt ist. Diese Buchse 36 ist mit einem Innengewinde versehen, mit dem der Gewindeabschnitt eines Kupplungsbolzens 37 in Eingriff steht, der gegen ein Drehen gesichert ist. Für diese Verdrehsicherung ist der Gewindeabschnitt des Kupplungsbolzens 37 mit einer Längsnut versehen, in die eine Nase einer Abdeckplatte 38 eingreift. Diese Nase ist am Rand einer Durchtrittsöffnung der Abdeckplatte 38 für den Kupplungsbolzen 37 vorgesehen.

Da der Kupplungsbolzen 37 nur eine Längsverschiebung und keine Rotation ausführen kann, ist die erforderliche Abdichtung der Halterung 35 gegen die Aufnahme 16 hin unproblematisch. Wie Figur 4 zeigt, ist für diese Abdichtung ein Faltenbalg 39 aus Gummi vorgesehen, der eine zentrale Öffnung für den Durchtritt des Kupplungsbolzens 37 hat. Diese zentrale Öffnung ist von einem Dichtungsring gebildet, der in eine Ringnut des Kupplungsbolzens 37 eingreift. Der äussere Rand des Faltenbalges 39 hat die Form eines umlaufenden Ringwulstes mit einer nach aussen offenen Ringnut. In diese greift eine Ringwulst der Aufnahme 16 ein.

Der mit dem Hohlzapfen 30 zu kuppelnde Endabschnitt des Kupplungsbolzens 37 ist mit einer Ringnut versehen, in welche zwei diametral angeordnete, gegeneinander weisende Klauen 40 eingreifen, welche von der Stirnseite des Hohlzapfens 30 abstehen. Bei der quer zur Längsrichtung des Hohlzapfens 30 erfolgenden Einschiebebewegung des Hohlkörpers 8 in die Aufnahme 16 schieben sich auch die beiden Klauen 40 in die zugeordnete Ringnut des Kupplungsbolzens 37. Das Kuppeln erfolgt deshalb selbsttätig beim Einsetzen des Hohlkörpers 8 in die Aufnahme 16, ebenso das Entkuppeln beim Herausnehmen aus der Aufnahme 16.

**Patentansprüche**

1. Vorrichtung zum Absaugen von Sekretflüssigkeit aus einer Wunde, in die ein Drain (1) eingeführt ist, der über einen Schlauch (2) an eine steuerbare Unterdruckquelle (5) angeschlossen ist, mit einem Sammelbehälter (3) für die abgesaugte Sekretflüssigkeit sowie einer Unterdruck-Messeinrichtung (7) und einem die Unterdruckquelle (5) steuernden Regler (10) mit Eingängen für den von der Unterdruck-Messeinrichtung (7) ermittelten Istwert sowie den vorgegebenen Sollwert des Unterdruckes, dadurch gekennzeichnet, dass ein Hohlkörper (8) mit veränderbarem Innenvolumen vorhanden ist, dessen Innenraum mit demjenigen des zum Drain (1) führenden Schlauches (2), in Verbindung steht, und dass der Drain (1), der Schlauch (2), der Sammelbehälter (3), die Unterdruck-Messeinrichtung (7) und der Hohlkörper (8) ein mikrobiologisch geschlossenes System bilden.

2. Vorrichtung nach Anspruch 1, dadurch gekennzeichnet, dass nur ein Sensorteil (7') der Unterdruck-Messeinrichtung (7) Bestandteil des mikrobiologisch geschlossenen Systemes ist.

3. Vorrichtung nach Anspruch 2, dadurch gekennzeichnet, dass die Unterdruckquelle durch einen Abschnitt (2') des vom Drain (1) zum Sammelbehälter (3) führenden Schlauches (2) und eine auf diesen Abschnitt einwirkende Schlauchpumpe (5) gebildet ist.

4. Vorrichtung nach Anspruch 2 oder 3, dadurch gekennzeichnet, dass der Sensorteil (7') der Unterdruck-Messeinrichtung ein aus Kunststoff bestehender Wegwerfteil mit zwei gegeneinander wirkenden Membranen (17, 21) unterschiedlicher Flächengrösse ist.

5. Vorrichtung nach Anspruch 4, dadurch gekennzeichnet, dass die beiden ringscheibenförmigen Membrane (17, 21) und einstückig mit ihnen ausgebildete Halter (18, 22) die beiden Stirnflächen eines dosenartigen Hohlkörpers bilden und dass der von der ringscheibenförmigen Membrane (21) mit dem kleineren Durchmesser umgebene, starre Zentralbereich an seiner Aussenseite mit einer Anlagefläche (23') für einen Messwandler (7'') versehen ist sowie auf seiner Innenseite über einen Distanzkörper (23) am starren Zentralbereich der anderen, im Durchmesser grösseren Membrane (17) abgestützt ist.

6. Vorrichtung nach einem der Ansprüche 3 bis 5, gekennzeichnet durch ein die Schlauchpumpe (5) enthaltendes Gehäuse (13) mit je einer Halterung (14, 15) für den der Einwirkung der Schlauchpumpe auszusetzenden Schlauchabschnitt (2') bzw. den Sensorteil (7') der Unterdruck-Messeinrichtung (7).

7. Vorrichtung nach einem der Ansprüche 1 bis 6, dadurch gekennzeichnet, dass die seitliche Begrenzungswand des Hohlkörpers (8) durch einen Faltenbalg (24) gebildet ist und die den Faltenbalg (24) an beiden Stirnseiten begrenzenden Verschlusskörper (25, 26) mittels einer Antriebsvorrichtung (9) in Balglängsrichtung relativ zueinander verstellbar sind.

8. Vorrichtung nach Anspruch 7, dadurch gekennzeichnet, dass die den Faltenbalg stirnseitig begrenzenden Verschlusskörper (25, 26) und der Faltenbalg (24) Teile einer Wegwerfeinheit sind, die in eine Halterung (16) des Gehäuses (13) einsetzbar ist, welches die Antriebsvorrichtung (9) enthält, und dass die Wegwerfeinheit über eine beim Einsetzen in die Halterung (16) in Eingriff und beim Herausnehmen ausser Eingriff kommenden Kupplung (40) mit der Antriebsvorrichtung (9) kuppelbar ist.

**Revendications**

1. Dispositif pour aspirer les sécrétions liquides d'une plaie dans laquelle est introduit un drain (1) qui est raccordé par un tuyau (2) à une source de dépression commandée (5), comprenant un récipient collecteur (3) destiné à recevoir les sécrétions liquides aspirées, ainsi qu'un dispositif de mesure de la dépression (7) et un régulateur (10) commandant la source de dépression (5) et présentant des entrées pour la valeur réelle mesurée par le dispositif de mesure de la dépression (7) ainsi que pour la valeur de consigne de la dépression, caractérisé en ce qu'il comprend un corps creux (8) à volume intérieur variable, dont l'espace intérieur est en communication avec celui du tuyau (2) menant au drain (1) et en ce que le drain (1), le tuyau (2), le récipient collecteur (3), le dispositif de mesure de la dépression (7) et le corps creux (8) forment ensemble un système fermé sous l'aspect microbiologique.

2. Dispositif selon la revendication 1, caractérisé en ce que seule une partie capteur (7') du dispositif de mesure de la dépression (7) fait partie du système microbiologique fermé.

3. Dispositif selon la revendication 2, caractérisé en ce que la source de dépression est formée par un segment (2') du tuyau (2) qui mène du drain (1) au récipient collecteur (3) et d'une pompe péristaltique (5) qui agit sur ce segment.

4. Dispositif selon la revendication 2 ou 3, caractérisé en ce que la partie capteur (7') du dispositif de mesure de la dépression est un organe usage unique composé de matière plastique, qui comprend deux membranes (17, 21) présentant des aires différentes et qui agissent l'une au sens inverse de l'autre.

5. Dispositif selon la revendication 4, caractérisé en ce que les deux membranes en forme de disque annulaire (17, 21), et des montures (18, 22) qui sont d'une seule pièce avec elles forment les deux surfaces frontales d'un corps creux en forme de boîte et en ce que la région centrale rigide, entourée par la membrane en forme de disque annulaire (21) qui possède le plus petit diamètre est munie sur son côté extérieur d'une surface d'appui (23') recevant un transducteur de mesure (7''), et est appuyée, sur son côté intérieur et par l'intermédiaire d'un élément entretoise (23), contre la région centrale de l'autre membrane (17) de plus grand diamètre.

6. Dispositif selon une des revendications 3 à 5, caractérisé par un boîtier (13) qui renferme la pompe péristaltique (5), et qui comprend deux montures (14, 15) respectivement pour le segment (2') du tuyau qui doit être soumis à l'action de la pompe péristaltique et pour la partie capteur (7') du dispositif de mesure de la dépression (7).

7. Dispositif selon une des revendications 1 à 6, caractérisé en ce que la paroi limite latérale du corps creux (8) est formée par un soufflet plissé (24) et les éléments de fermeture (25, 26) qui limitent le soufflet plissé (24) sur les deux faces frontales peuvent être déplacés l'un par rapport à l'autre dans la direction longitudinale du soufflet au moyen d'un dispositif moteur (9).

8. Dispositif selon la revendication 7, caractérisé en ce que les éléments de fermeture (25, 26) qui limitent le soufflet plissé au niveau de ses faces frontales et le soufflet plissé (24) font partie d'une unité à usage unique qui peut être emboîtée dans une monture (16) du boîtier (13) qui renferme le dispositif moteur (9) et en ce que l'unité à usage unique peut être accouplée au dispositif moteur (9) par l'intermédiaire d'un accouplement (40) qui entre en prise lors de l'engagement dans la monture (16) et se met hors de prise lors de l'extraction.

**Claims**

1. A device for the vacuum extraction of secreted fluid from a wound, into which a drain (1) is introduced, the said drain being connected by a tube (2) to a controllable source (5) of negative pressure, and comprising a collecting tank (3) for the extracted secreted fluid and with a negative pressure measuring device (7) and, for controlling the source (5) of negative pressure, a regulator (10) with inputs for the actual value ascertained by the negative pressure measuring device (7) and for the preset desired value of the negative pressure, characterised in that a hollow body (8) of variable internal volume is provided, the interior of which is connected to that of the tube (2) which leads to the drain (1) and in that the drain (1), the tube (2), the collecting tank (3), the negative pressure measuring device (7) and the hollow body (8) constitute one microbiologically closed system.

2. A device according to Claim 1, characterised in that only one sensor part (7') of the negative pressure measuring device (7) is a constituent part of the microbiologically closed system.

3. A device according to Claim 2, characterised in that the source of negative pressure is constituted by a portion (2') of the tube (2) leading from the drain (1) to the collecting tank (3) and a hose pump (5) which acts upon this portion.

4. A device according to Claims 2 or 3, characterised in that the sensor part (7') of the negative pressure measuring device is a disposable part which consists of synthetic plastics material and which has two oppositely acting diaphragms (17, 21) of different surface sizes.

5. A device according to Claim 4, characterised in that the two diaphragms (17, 21) which are in the form of annular discs, and holders (18, 22) which are constructed in one piece with them form the two end faces of a can-like hollow body and in that the rigid central portion which is surrounded by the annular disc-shaped diaphragm (21) which is of the smaller diameter is provided on its outside with a bearing surface (23') for a transducer (7'') while on its inside face it is braced via a spacer (23) on the rigid central area of the other larger-diameter diaphragm (17).

6. A device according to one of Claims 3 to 5, characterised by a housing (13) containing a hose pump (5), with respective holders (14, 15) for the portion of tube (2') which is to be subject to the action of the hose pump and for the sensor part (7') of the negative pressure measuring device (7).

7. A device according to one of Claims 1 to 6, characterised in that the lateral boundary wall of the hollow body (8) consists of a bellow (24) and in that the closure members (25, 26) which bound the bellows (24) at both ends can be adjusted relatively to each other in the longitudinal direction of the bellows by means of a drive device (9).

8. A device according to Claim 7, characterised in that the closure members (25, 26) which define the ends of the bellows, and the bellows (24), are parts of a disposable unit which can be inserted into a holder (16) in the housing (13) which contains the drive device (9) and in that the disposable unit is adapted to be coupled to the drive device (9) via a coupling (40) which becomes engaged upon insertion into the holder (16) and disengaged upon removal therefrom.

½

Fig.1

Fig.2

## Fig. 3

## Fig. 4